Europäisches Patentamt

European Patent Office

Office européen des brevets

(19)

(11) Veröffentlichungsnummer: **0 175 671**
**A1**

(12) ## EUROPÄISCHE PATENTANMELDUNG

(21) Anmeldenummer: 85890111.9

(22) Anmeldetag: 15.05.85

(51) Int. Cl.⁴: **A 61 K 31/44,** A 61 K 9/12

(30) Priorität: 23.08.84 AT 2708/84

(43) Veröffentlichungstag der Anmeldung: 26.03.86
Patentblatt 86/13

(84) Benannte Vertragsstaaten: BE CH DE FR GB IT LI LU NL
SE

(71) Anmelder: Kuhlemann & Co., Salmgasse 4, A-1030 Wien
(AT)

(72) Erfinder: Burghart, Walter, Salmgasse 4, A-1030 Wien
(AT)

(74) Vertreter: Haffner, Thomas M., Dr. et al,
Patentanwaltskanzlei Dipl.-Ing. Adolf Kretschmer Dr.
Thomas M. Haffner Schottengasse 3a, A-1014 Wien (AT)

(54) **Pharmazeutische Zubereitung sowie Verfahren zum Darreichen dieser pharmazeutischen Zubereitung.**

(57) Die pharmazeutische Zubereitung mit Nifedipin als
Wirkstoff wird als Aerosol entweder unter Verwendung eines
Treibgases oder mittels eines Pumpensprays verabreicht.
Bei Verwendung von Treibgasen enthält die Zubereitung bevorzugt 2 bis 5 Gew.% Nifedipin, 25 bis 40 Gew.% Polyäthylenglykol, 40 bis 25 Gew.% Äthanol und 30 bis 40 Gew.%,
vorzugsweise etwa 35 Gew.% Treibmittel, wohingegen im
Falle eines Pumpensprays die Zubereitung mit in vorteilhafter Weise 10 mg Nifedipin, 70 bis 100 mg Polyäthylenglykol,
insbesondere ein Gemisch von Polyäthylenglykol 600 und
Polyäthylenglykol 400 im Verhälnis 5 zu 4 bis 4 zu 5, 30 bis
40 mg Äthanol, 5 bis 20 mg Wasser und ggf. 2 bis 5 mg Saccharin und ggf. 1 bis 3 mg Geschmacksstoffe, wie Pfefferminzöl.

EP 0 175 671 A1

## Pharmazeutische Zubereitung sowie Verfahren zum Darreichen dieser pharmazeutischen Zubereitung

Die Erfindung bezieht sich auf eine pharmazeutische Zubereitung mit Nifedipin als Wirkstoff. Nifedipin ist als Koronartherapeutikum bekannt und Nifedipin enthaltende feste Präparatzusammensetzungen sowie Verfahren zu ihrer Herstellung sind beispielsweise aus der DE-OS 28 22 882 bekannt geworden. Bei Nifedipin handelt es sich um 4-(2-nitrophenyl)--2,6-dimethyl-3,5-dicarbomethoxi-1,4-dihydropyridin und es ist weiters bereits bekannt, daß diese Verbindung in hohem Maße lichtempfindlich ist. Es sind daher bereits eine Reihe von Vorschlägen gemacht worden, wie die Verabreichung dieser Verbindung erfolgen soll, um die mit der Zersetzung unter Lichteinfluß verbundene Abnahme der Wirksamkeit bzw. den Verlust an Wirksamkeit zu beseitigen. Im besonderen ist aus der DE-OS 28 22 882 eine Präparatzusammensetzung zu entnehmen, bei welcher Nifedipin als feste Präparatzusammensetzung mit Polyvenylpyrolidonmethylzellulose, Hydroxypropylzellulose und Hydroxypropylmethylzellulose vermengt wird, wobei zusätzlich verschieden oberflächenaktive Mittel, insbesondere Öle, angewandt wurden. Diese Verabreichungsform wurde in erster Linie im Hinblick auf die Verbesserung der Verfügbarkeit des Arzneistoffes nach der Resorption gewählt und ist weiterhin mit erheblichen Nachteilen bezüglich der Stabilität unter Lichteinfluß verbunden. Zur oralen Verabreichung sind neben Tabletten und Pillen auch schon Kapseln vorgeschlagen worden, wobei bei diesen Kapseln Farbstoffe in die Kapselhülle einzuarbeiten sind, um die Lichtzersetzung hintanzuhalten. Eine derartige Zusammensetzung ist beispielsweise der DE-PS 22 09 526 zu entnehmen, wobei als Farbstoff Gelb-Orange S 15985 neben einem Opakisierungsmittel in der Kapselhülle gewählt wurde. Derartige Kapselhüllen sind in der Regel temperaturempfindlich, und es ist auch in diesem Fall eine vollständige Sicherheit gegen Zersetzung der wirksamen Substanz nicht gegeben.

Die Erfindung zielt nun darauf ab, eine pharmazeutische Zubereitung der eingangs genannten Art zu schaffen, bei welcher ein hohes Maß an Sicherheit in bezug auf die Zersetzung der wirksamen Substanz erzielt wird und welche es ermöglicht, die Wirkungsdauer bzw. die Geschwindigkeit des Ansprechens des Medikamentes in weiten Grenzen einzustellen. Zur Lösung dieser Aufgabe besteht die Erfindung im wesentlichen darin, daß der Wirkstoff gegebenenfalls gemeinsam mit pharmazeutischen Hilfsstoffen, insbesondere Aromastoffen, Weichmachern, Lösungsmitteln oder Beschichtungsmitteln als Aerosol vorliegt. Dadurch, daß der Wirkstoff gegebenenfalls gemeinsam mit pharmazeutischen Hilfsstoffen, insbesondere Aromastoffen, Weichmachern, Lösungsmitteln oder Beschichtungsmitteln, als Aerosol vorliegt, kann der Wirkstoff in einer lichtdichten Verpackung vorliegen, und im Falle eines unbeschichteten Versprühens unmittelbar und rasch zur Wirkung gelangen. Die Resorption der Aerosols bei inhalatorischer oder sublingualer Anwendung kann überaus rasch erfolgen, und die Anwesenheit von Beschichtungsmitteln erlaubt es, einen gewissen Anteil von beschichteten Wirkstoff enthaltenden Teilchen abzugeben, welche erst verzögert zur Wirkung gelangen. Die Verpackung in lichtdichten Behältern wie beispielsweise Aerosoldosen, welche unter Treibgasdruck stehen können oder aber im Falle der Anwendung einer Pumpwirkung ein Versprühen mit atmosphärischer Luft als Trägergas ermöglichen, erlaubt hiebei den Wirkstoff auch bei Temperaturschwankungen stabil zu halten und erleichtert die praktische Anwendung wesentlich.

Insbesondere für inhalatorische Anwendung ist die pharmazeutische Zubereitung mit Vorteil so gebildet, daß der Wirkstoff in fester Suspension im Trägergasstrom bzw. Treibgasstrom vorliegt und mit einem Lösungsvermittler, wie Polyäthylenglykol, vermengt und/oder ummantelt ist. Als Trägergasstrom kommen übliche inerte Trägergase bzw. Treibgase, wie z.B. halogenierte Kohlenwasserstoffe in Frage. Im

- 3 -

Falle einer Aerosoldose mit eingebauter Pumpe läßt sich als Trägergas ohne weiters atmosphärische Luft verwenden, wobei aufgrund der raschen Resorption nach der Abgabe durch die Aerosol versprühende Düse oxydative Zersetzungen nicht zu befürchten sind. Die Lagerung des Wirkstoffes unter inertem Trägergasdruck trägt weiter zur Langzeitstabilität des Wirkstoffes bei.

In besonders einfacher Weise ist der Wirkstoff in einem pharmazeutisch unbedenklichen Lösungsmittel, insbesondere Polyalkoholen, gelöst und als Lösung im Trägergasstrom suspendiert. Durch Wahl eines geeigneten unbedenklichen Lösungsmittels läßt sich auch mit relativ einfachen Einrichtungen die gewünschte Dosierung sicherstellen. Mit besonderem Vorteil ist jedoch der Wirkstoff in pharmazeutisch verträglichen, filmbildenden flüssigen Polymeren, insbesondere Polymethacrylaten, gelöst und gegebenenfalls mit Lichtschutzpigmenten wie z.B. gefärbtes $TiO_2$, $Fe_xO_y$ versetzt und in dieser Lösung im Trägergasstrom suspendiert. Ein filmbildendes flüssiges Polymer erlaubt es, den Wirkstoff bei seinem Versprühen zu umhüllen, so daß ein Teil der Wirkstoff enthaltenden Teilchen gekapselt verabreicht wird und somit verzögert zur Wirkung gelangt. Wenn eine transdermale Applikation angestrebt wird, ist es hiebei erforderlich, die filmbildenden flüssigen Polymere mit Lichtschutzpigmenten zu versehen, um eine Zersetzung während der Dauer der Resorption durch die Haut zu verhindern. In vorteilhafter Weise liegt für die Erzielung einer raschen Ansprechzeit und einer Langzeitwirkung der Wirkstoff zu wenigstens 10 Gew% bezogen auf das Gesamtgewicht des Wirkstoffes als ummantelte Teilchen vor, deren Mantel aus pharmazeutisch verträglichem Material, insbesondere Kunststoffen wie Polyäthylen oder Hydroxypropylmethylzellulosephthalat besteht, welche als Suspension im Trägerstrom vorliegen.

Als Trägergasstrom kann, wie bereits erwähnt, in bekannter Weise Treibgas auf der Basis fluorierter Kohlenwasserstoffe oder aber komprimierte Luft verwendet werden.

Eine geeignete Dosis für verschiedene Formen der Anwendung läßt sich dadurch erzielen, daß die Wirkstoffdosis bei Suspension als Feststoff für inhalatorische Anwendung je Applikation mit 0,5 - 5 mg, für sublinguale Anwendung, insbesondere in Lösung und/oder als beschichtete Teilchen mit 2 - 20 mg und für transdermale Applikation mit 20 - 100 mg je Applikation gewählt wird.

In besonders einfacher Weise läßt sich ein derartiges Präparat dadurch verabreichen, daß die Wirksubstanz mit einem pharmazeutisch unbedenklichen gasförmigen Träger versprüht wird. Für diese Form der Verabreichung können konventionelle Einrichtungen, wie sie beispielsweise für die Verabreichung von Nitroglycerin als Aerosol bereits bekannt geworden sind, eingesetzt werden, wobei lediglich eine geeignete Dosierung sichergestellt werden muß.

Bei der Verwendung von Treibmittel, beispielsweise von halogenierten Kohlenwasserstoffen als Treibmittel, sind jedoch eine Reihe von Randbedingungen zu beachten, um sicherzustellen, daß ein Ausfällen von Nifedipin oder eine Phasentrennung verhindert wird. Eine besonders bevorzugte pharmazeutische Zubereitung für eine reproduzierbare Dosierung und die Anwendung von Treibgas enthält daher 2 bis 5 Gew.% Nifedipin, 25 bis 40 Gew.% Polyäthylenglykol, 40 bis 25 Gew.% Äthanol und 30 bis 50 Gew.%, vorzugsweise etwa 35 Gew.% Treibmittel.

Ein wesentliches Erfordernis für die Durchführung des erfindungsgemäßen Verfahrens besteht hiebei darin, daß es sich bei dem Behältnis, welches für die Bereitstellung der Wirksubstanz eingesetzt wird, um ein lichtgeschütztes bzw.

lichtdichtes Behältnis handelt, und es wird vorzugsweise das Verfahren so durchgeführt, daß die Wirksubstanz in stabiler Lösung in ein lichtgeschütztes Behältnis eingebracht ist und über eine Dosiervorrichtung in den Ausstoßkanal gebracht wird. Um eine zumindest teilweise Ummantelung der Teilchen zu erzielen wird vorzugsweise so vorgegangen, daß die Wirksubstanz gemeinsam mit einem filmbildenden flüssigen bzw. gelösten Polymer, insbesondere PMMA oder HPMCP, mit Trägergas versprüht wird. Bei Verwendung von Polymethylmetacrylat oder Hydroxypropylmethylzellulosephthalat in flüssiger Form wird ein Teil der beim Versprühen ausgestoßenen Teilchen nach dem Verlassen der Sprühdüse ummantelt und durch Wahl des Anteiles an diesem filmbildenden, flüssigen Polymer und Wahl geeigneter Austrittsöffnungen für die Düse läßt sich der Anteil der ummantelten Teilchen im Aerosol in weiten Grenzen einstellen. Eine größere Menge an ummantelten Teilchen führt hiebei zur Verlängerung der Langzeitwirkung, wohingegen ein Versprühen in nichtummantelter Form für eine akute Behandlung vorteilhaft ist.

Für das Ausbringen der pharmazeutischen Zubereitung mittels eines Pumpensprays muß eine entsprechende Viskosität sichergestellt werden, um reproduzierbar gleiche Mengen des Wirkstoffes ausbringen zu können. Für die Verwendung als Pumpenspray enthält die pharmazeutische Zubereitung bevorzugt 5 bis 20 mg Nifedipin, 70 bis 300 mg Polyäthylenglykol, insbesondere ein Gemisch von Polyäthylenglykol 400 im Verhältnis 5 zu 4 bis 4 zu 5, 30 bis 135 mg Äthanol, 5 bis 20 mg Wasser und ggf. 3 bis 20 mg Glycerin sowie ggf. 2 bis 20 mg Saccharin und ggf.1 bis 12 mg Geschmacksstoffe, Pfefferminzöl, enthält.

Die Erfindung wird nachfolgend anhand von Ausführungsbeispielen näher erläutert.

0175671

Beispiel 1:

Es wurde ein Gewichtsteil Nifedipin mit 15 - 35 Gewichtsteilen Polyäthylenglykol mit einem Durchschnittsmolekulargewicht von 300 - 600 unter Erwärmen gelöst und in eine Aluminiumdose abgefüllt. Die Dose wurde mit einem Pumpdosierventil verschlossen, dessen Ansaugkanal in die Flüssigkeit eintaucht. Mit einem Pumpenhub wurden 4 mg Nifedipin als Aerosol ausgestoßen und sublingual zur Anwendung gebracht. Die Lösung erwies sich als stabil, entsprach aber geschmacklich nicht.

Beispiel 2:

Für ein Pumpenspray mit einer Dosierpumpe für ungefähr 150 mg wurden eine Reihe von Mischungen bereitet, wobei neben dem Einsatz von PEG 400 in Mischung mit PEG 600 zur Absenkung der Viskosität Alkohol und Wasser zugesetzt wurde. Eine gute Sprühbarkeit und reproduzierbare Dosierbarkeit wurde innerhalb der nachfolgenden Bereiche für 150 mg je Applikation festgestellt:

10 mg Nifedipin, 70 bis 100 mg Polyäthylenglykol, insbesondere ein Gemisch von Polyäthylenglykol 600 und Polyäthylenglykol 400 im Verhältnis 5 zu 4 bis 4 zu 5, 30 bis 40 mg Äthanol, 5 bis 20 mg Wasser und ggf. 3 bis 5 mg Glycerin sowie ggf. 2 bis 5 mg Saccharin und ggf. 1 bis 3 mg Geschmacksstoffe, wie Pfefferminzöl.

Beispiel 3:

Für die Herstellung eines Treibgassprays wurden eine Reihe verschiedener Mischungen erprobt. Bei einer größeren Anzahl derartiger Formulierungen wurde beobachtet, daß Nifedipin auskristallisiert oder eine Phasentrennung bzw. Entmischung auftrat. Eine stabile und reproduzierbar zu dosierende Zubereitung konnte nur innerhalb der nachfolgenden Bereichsgrenzen gefunden werden:

0175671

2 bis 5 Gew.% Nifedipin, 25 bis 40 Gew.% Polyäthylenglykol, 40 bis 25 Gew.% Äthanol und 30 bis 50 Gew.%, vorzugsweise etwa 35 Gew.% Treibmittel.

Beispiel 4:

Für die Herstellung eines Pumpensprays mit einer Dosierpumpe mit ungefähr 220 mg wurde 10 mg Nifedipin in 66 mg Polyäthylenglykol 600 und 47 mg Polyäthylenglykol 400 gelöst. Es wurden 56 mg Äthanol zugesetzt. Weiters wurde 6 mg Glycerin, 21 mg Wasser, 4,5 mg Natriumsaccharat und 3,5 mg Pfefferminzöl eingesetzt.

Ein Pumpenspray dieser Zusammensetzung zeigte gegenüber den meisten Pumpensprays entsprechend Beispiel 2 eine verbesserte Tieftemperaturbeständigkeit und es wurde auch bei extrem tiefen Temperaturen ein Ausfallen von Nifedipin aus dem Lösungsmittel nicht beobachtet.

Als Treibgase können im Rahmen der vorliegenden Erfindung neben den bereits erwähnten fluorierten Halogenkohlenwasserstoffen, Dimethyläther und im speziellen Dichlorfluormethan, Trichlorfluormethan, 1,1,2,2-Dichlortetrafluoräthan und Mischungen derselben eingesetzt werden.

Anstelle der vorgeschlagenen Polyäthylenglykole, insbesondere der Mischungen aus Polyäthylenglykol 400 und 600 lassen sich auch partielle Fettsäureester des Sorbitans bzw. des Polyhydroxiäthylensorbitans, sowie Polyvinylpyrolidone, Polyvinylalkohole, Polyhydroxyäthylenfettalkoholäther bzw.Polyhydroxyäthylenfettsäureester sowie Polyhydroxyäthylen-polyhydroxypropylenkondensate verwenden. Auch äthoxylierte Triglyceride eignen sich als Lösungsmittel für den Wirkstoff. Im besonderen können auch Polyroxyäthylenfettalkoholäther, Polyoxyäthylensorbitanfettsäureester oder Polyoxyäthylenstearinsäureester zu diesen Zwecken herangezogen werden.

0175671

- 8 -

Weiters können neben Süßstoffen (Natriumsaccharat auch Aromastoffe und gegebenenfalls auch Farbstoffe, wie z.B. Curcumin oder auch ätherische Öle (Pfefferminzöl) eingesetzt werden.

Patentansprüche:

1. Pharmazeutische Zubereitung mit Nifedipin als Wirkstoff, dadurch gekennzeichnet, daß der Wirkstoff gegebenenfalls gemeinsam mit pharmazeutischen Hilfsstoffen, insbesondere Aromastoffen, Weichmachern, Lösungsmitteln oder Beschichtungsmitteln als Aerosol vorliegt.

2. Pharmazeutische Zubereitung nach Anspruch 1, dadurch gekennzeichnet, daß der Wirkstoff in fester Suspension im Trägergasstrom vorliegt.

3. Pharmazeutische Zubereitung nach Anspruch 1 oder 2, dadurch gekennzeichnet, daß der Wirkstoff in einem pharmazeutisch unbedenklichen Lösungsmittel, insbesondere Polyalkoholen, gelöst und als Lösung im Trägergasstrom bzw. Treibgasstrom suspendiert ist.

4. Pharmazeutische Zubereitung nach Anspruch 1, 2 oder 3, dadurch gekennzeichnet, daß der Wirkstoff in pharmazeutisch verträglichen, filmbildenden flüssigen Polymeren, insbesondere Polymethacrylaten, gelöst und gegebenenfalls mit Lichtschutzpigmenten wie z.B. gefärbtes $TiO_2$, $Fe_xO_y$ versetzt ist und in dieser Lösung im Trägergasstrom suspendiert ist.

5. Pharmazeutische Zubereitung nach einem der Ansprüche 1 bis 4, dadurch gekennzeichnet, daß der Wirkstoff zu wenigstens 10 Gew% als ummantelte Teilchen vorliegt, deren Mantel aus pharmazeutisch verträglichem Material, insbesondere Kunststoffen wie Polyäthylen oder Hydroxypropylmethylzellulosephthalat besteht, welche als Suspension im Trägerstrom vorliegen bzw. für inhalatorische Zwecke mit einem Lösungsvermittler, wie Polyäthylenglykol vermengt und/oder ummantelt ist.

6. Pharmazeutische Zubereitung nach einem der Ansprüche 1 bis 5, dadurch gekennzeichnet, daß der Trägergasstrom von komprimierter Luft gebildet ist.

7. Pharmazeutische Zubereitung nach einem der Ansprüche 1 bis 6, dadurch gekennzeichnet, daß die Wirkstoffdosis bei Suspension als Feststoff für inhalatorische Anwendung je Applikation mit 0,5 - 5 mg, für sublinguale Anwendung insbesondere in Lösung und/oder als beschichtete Teilchen mit 2 - 20 mg und für transdermale Applikation mit 20 - 100 mg je Applikation gewählt wird.

8. Pharmazeutische Zubereitung nach Anspruch 1, dadurch gekennzeichnet, daß sie 2 bis 5 Gew.% Nifedipin, 25 bis 40 Gew.% Polyäthylenglykol, 40 bis 25 Gew.% Äthanol und 30 bis 50 Gew.%, vorzugsweise etwa 35 Gew.% Treibmittel, enthält.

9. Pharmazeutische Zubereitung nach Anspruch 6, dadurch gekennzeichnet, daß sie in 100 bis 500 mg Spray, 5 bis 20 mg Nifedipin, 70 bis 100 mg Polyäthylenglykol, insbesondere ein Gemisch von Polyäthylenglykol 600 und Polyäthylenglykol 400 im Verhältnis 5 zu 4 bis 4 zu 5, 30 bis 135 mg Äthanol, 5 bis 70 mg Wasser und ggf. 3 bis 20 mg Glycerin sowie ggf. 2 bis 5 mg Saccharin und ggf. 1 bis 12 mg Geschmacksstoffe, wie Pfefferminzöl, enthält.

10. Verfahren zum Darreichen von pharmazeutischen Zubereitungen nach einem der Ansprüche 1 bis 9, dadurch gekennzeichnet, daß die Wirksubstanz mit einem pharmazeutisch unbedenklichen gasförmigen Träger versprüht wird.

11. Verfahren nach Anspruch 10, dadurch gekennzeichnet, daß die Wirksubstanz in stabiler Lösung in ein licht-

0175671

geschütztes Behältnis eingebracht ist und über eine Dosiervorrichtung in den Ausstoßkanal gebracht wird.

12. Verfahren nach Anspruch 10 oder 11, dadurch gekennzeichnet, daß die Wirksubstanz gemeinsam mit einem filmbildenden flüssigen bzw. gelösten Polymer, insbesondere PMMA oder HPMCP, mit Trägergas versprüht wird.

**Europäisches Patentamt**

**EUROPÄISCHER RECHERCHENBERICHT**

0175671

Nummer der Anmeldung

EP 85 89 0111

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | CHEMICAL ABSTRACTS, Band 97, Nr. 3, 19. Juli 1982, Seite 58, Nr. 16919v, Columbus, Ohio, US; P.E. MALO et al.: "Effects of intravenous and aerosol nifedipine on prostaglandin F2alpha and histamine-induced bronchoconstriction in anesthetized dogs" & J. PHARMACOL. EXP. THER. 1982, 221(2), 410-15 | 1-12 | A 61 K 31/44 A 61 K 9/12 |
| X,Y | CHEMICAL ABSTRACTS, Band 98, Nr. 17, 25. April 1983, Seite 47, Nr. 137459z, Columbus, Ohio, US; T.M. BRUGMAN et al.: "Nifedipine aerosol attenuates airway constriction in dogs with hyperreactive airways" & AM. REV. RESPIR. DIS. 1983, 127(1), 14-17 | 1-12 | |
| X | CHEMICAL ABSTRACTS, Band 97, Nr. 10, 6. September 1982, Seite 423, Nr. 78923a, Columbus, Ohio, US; & JP - A - 82 85 316 (KANEBO, LTD.) 28-05-1982 * Zusammenfassung * | 1,3,4, 6 | RECHERCHIERTE SACHGEBIETE (Int. Cl.4) A 61 K |
| Y | CHEMICAL ABSTRACTS, Band 98, Nr. 26, 27. Juni 1983, Seite 383, Nr. 221832y, Columbus, Ohio, US; & JP - A - 58 46 019 (KANEBO, LTD.) 17-03-1983 | 5 | |

--- -/-

Der vorliegende Recherchenbericht wurde für alle Patentansprüche erstellt.

| Recherchenort DEN HAAG | Abschlußdatum der Recherche 02-12-1985 | Prüfer BENZ K.F. |
|---|---|---|

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 004 650 (BAYER AG) * Seite 1, Zeilen 1-17; Seite 6, Zeile 3; Seite 7, Zeilen 1-13; Seiten 10,11, Beispiele 3,4 * | 1,3 | |
| Y | LU-A- 65 929 (BAYER AG) * Insgesamt * & DE - A - 2 209 526 (Kat. D,Y,) | 4,7-9, 11 | |
| Y | GB-A- 970 027 (REVLON) * Insgesamt und insbesondere Seite 2, Zeilen 104-105, 108-109 * | 1,10 | |
| Y | EP-A-0 055 397 (BAYER AG) * Seite 2, Zeilen 7-30; Seite 14, Zeilen 21-27 * | 4,6,10 ,12 | **RECHERCHIERTE SACHGEBIETE (Int. Cl.4)** |
| Y | JOURNAL OF PHARMACEUTICAL SCIENCES, Band 64, Nr. 3, März 1975, Seiten 408-14, Washington, D.C., US; W. FEINSTEIN et al.: "Development and evaluation of a dexamethasone timed-release aerosol formulation" * Seite 408; Seite 413, Ansprüche * | 5 | |

Der vorliegende Recherchenbericht wurde für alle Patentansprüche erstellt.

| Recherchenort | Abschlußdatum der Recherche | Prüfer |
|---|---|---|
| DEN HAAG | 02-12-1985 | BENZ K.F. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82